# DEMANDE DE BREVET EUROPEEN

(11) **EP 0 792 651 A2**
(43) Date de publication de la demande: **03.09.1997**
(21) Numéro de dépôt: 97400395.6
(22) Date de dépôt: 24.02.1997
(51) Int. Cl.: A61L 2/18, G02C 13/00

(54) **Ensemble et procédé de désinfection de lentilles de contact**

(30) Priorité: 29.02.1996 FR 9602563
(71) Demandeur: ESSILOR INTERNATIONAL (COMPAGNIE GENERALE D'OPTIQUE), F-94227 Charenton cédex (FR)
(72) Inventeur: Bourset, Claude, 94000 Créteil (FR); Comte, Laurent, 75015 Paris (FR); Soyer, Patrice, 77390 Guignes (FR)
(74) Mandataire: Casalonga, Axel

(57) **Abrégé**

L'ensemble comprend un corps d'étui (10), un couvercle (20) amovible et un support de lentilles de contact (40). Un élément catalytique (50) est disposé sur la surface interne du corps d'étui (10). Un repère visuel (15) est ménagé sur le corps d'étui (10) en dessous de l'élément catalytique (50) pour définir un volume déterminé de solution de désinfection à introduire initialement dans le corps d'étui. Le support de lentilles de contact (40) est conçu de telle sorte que lors de son introduction dans le corps d'étui (10), il déplace un volume déterminé de solution oxydante suffisant pour recouvrir en totalité l'élément catalytique (50) et les lentilles de contact à désinfecter.

Application : la désinfection de lentilles de contact au moyen d'une solution oxydante à base de peroxyde d'hydrogène.

## Description

La présente invention concerne d'une manière générale un ensemble de désinfection de lentilles de contact, et plus particulièrement un tel ensemble dans lequel les lentilles de contact sont désinfectées au moyen d'une solution de désinfection, par exemple une solution oxydante à base de peroxyde d'hydrogène, dans lequel la désinfection des lentilles de contact et la neutralisation de la solution de désinfection s'effectuent simultanément.

La présente invention concerne également un procédé de mise en oeuvre de désinfection de lentilles de contact à l'aide d'un ensemble de désinfection permettant la désinfection de lentilles de contact à l'aide d'une solution de désinfection et simultanément la neutralisation de cette solution.

Il est connu, pour la désinfection de lentilles de contact, d'immerger les lentilles de contact dans une solution de désinfection oxydante à base de peroxyde d'hydrogène.

Il va de soi qu'il est extrêmement dangereux d'insérer dans l'oeil une lentille de contact venant juste d'être retirée d'une solution désinfectante de peroxyde d'hydrogène, car une telle solution désinfectante est fortement irritante pour l'oeil. Par conséquent, la solution désinfectante de peroxyde d'hydrogène doit être neutralisée avant la réutilisation des lentilles de contact. Cette étape de neutralisation s'effectue de façon commode en mettant en contact un catalyseur de décomposition du peroxyde d'hydrogène avec la solution de peroxyde d'hydrogène.

On a également proposé des procédés et des ensembles de désinfection de lentilles de contact dans lesquels on mettait simultanément en contact avec une solution de désinfection, telle qu'une solution de peroxyde d'hydrogène, les lentilles de contact et le catalyseur de neutralisation de telle sorte que la réaction de neutralisation de la solution de désinfection commençait en même temps que la décontamination des lentilles de contact .

Le document EP-A-354 876 décrit un procédé et un ensemble de désinfection de lentilles de contact dans lesquels la désinfection des lentilles de contact et la neutralisation de la solution de désinfection s'effectuent simultanément. L'ensemble de désinfection du document EP-A-354 876 comprend un récipient et un couvercle détachable monté sur le récipient. Un support de lentilles de contact et de bloc de catalyseur solidaire du bouchon pend à partir de ce couvercle dans le récipient. Avec cet ensemble de désinfection, la solution de désinfection, par exemple une solution de peroxyde d'hydrogène, est introduite dans le récipient, puis le bloc de catalyseur et les lentilles de contact sont introduits simultanément dans la solution lorsque l'on fixe le couvercle sur l'extrémité du récipient. Dans une réalisation du document EP-A-354 876, le bloc de catalyseur est contenu dans un logement réalisé dans le support de lentilles de contact sur un côté des paniers destinés à maintenir les lentilles de contact. Une telle réalisation conduit à un support de lentilles de contact et de bloc de catalyseur complexe.

Dans une autre réalisation du document EP-A-354 876, le bloc de catalyseur est simplement monté à l'extrémité inférieure du support de lentilles de contact, en dessous des paniers de maintien des lentilles de contact. Avec une telle construction du support de lentilles de contact et de bloc de catalyseur, le bloc de catalyseur se trouve exposé à un contact accidentel lorsque le couvercle et le support de lentilles de contact et de bloc de catalyseur sont retirés du récipient.

Dans le document EP-A-560 728, on décrit un ensemble de désinfection de lentilles de contact comprenant un corps d'étui et un couvercle auquel se trouve fixé un support de lentilles de contact. Un catalyseur est disposé dans la partie supérieure du corps d'étui sur la surface interne de celui-ci. Un agitateur, par exemple un agitateur magnétique à ailette, est disposé au fond du corps d'étui. Lors de l'utilisation, on remplit le corps d'étui avec une solution oxydante de peroxyde d'hydrogène jusqu'à un niveau situé au-dessus du catalyseur, puis on introduit le support de lentilles de contact contenant les lentilles de contact à désinfecter en fixant le couvercle sur l'extrémité ouverte du corps d'étui. Enfin, on agite la solution de peroxyde en mettant en rotation l'agitateur.

Dans cette réalisation du document EP-A-560 728, la solution oxydante de désinfection est initialement introduite jusqu'à remplir le corps d'étui au-dessus du catalyseur, de sorte que la réaction de neutralisation de la solution oxydante débute avant que le support de lentilles de contact, et par conséquent les lentilles de contact à désinfecter, ne soit introduit dans le corps d'étui. De ce fait, il existe un risque que la solution oxydante soit neutralisée avant l'introduction du support de lentilles de contact et des lentilles de contact à désinfecter, à un point tel que la solution oxydante de désinfection n'ait plus l'efficacité suffisante pour désinfecter de façon appropriée les lentilles de contact.

La présente invention a donc pour objet de fournir un ensemble de désinfection de lentilles de contact dans lequel la désinfection des lentilles de contact s'effectue simultanément avec la neutralisation de la solution de désinfection, qui soit de construction simple, qui évite tout risque de contact accidentel du catalyseur, en particulier avec les doigts d'un utilisateur ou tout autre objet, et qui assure que la neutralisation de la solution de désinfection ne débute que lorsque les lentilles de contact sont immergées dans la solution de désinfection.

La présente invention a également pour objet un procédé de mise en oeuvre d'un ensemble tel que défini ci-dessus.

On atteint les buts ci-dessus, selon l'invention, en réalisant un ensemble de désinfection de lentilles de contact au moyen d'une solution de désinfection, par exemple une solution oxydante à base de peroxyde d'hydrogène, comprenant :
- un étui constitué d'un corps d'étui, de préférence transparent ou translucide, ouvert à une extrémité, ayant une paroi de fond et une paroi latérale, et d'un couvercle amovible destiné à être fixé sur l'extrémité ouverte du corps d'étui;
- un support de lentilles de contact destiné à être introduit jusqu'à une position finale à l'intérieur du corps d'étui; et
- un élément catalytique de neutralisation de la solution de désinfection disposé sur la surface interne de la paroi latérale du corps d'étui à une distance prédéterminée de la paroi de fond du corps d'étui, caractérisé en ce qu'il comprend en outre un repère visuel sur la paroi latérale du corps d'étui disposé à une distance prédéterminée en dessous de l'élément catalytique et définissant un volume prédéterminé de solution de désinfection à introduire initialement dans le corps d'étui et en ce que le support de lentilles de contact est conçu de telle sorte que lorsque le support de lentilles de contact introduit dans le corps d'étui est dans sa position finale, le volume de solution de désinfection déplacé par le support de lentilles de contact est suffisant pour recouvrir, de préférence entièrement, l'élément catalytique et les lentilles de contact à désinfecter.

De préférence, le support de lentilles de contact est solidaire du couvercle et saille à partir de ce couvercle de manière à pénétrer à l'intérieur du corps d'étui, jusqu'à sa position finale, lorsque le couvercle est fixé sur l'extrémité ouverte du corps d'étui.

Le couvercle et le support de lentilles peuvent former un sous-ensemble monobloc obtenu, par exemple, par moulage par injection.

Lorsque le support de lentilles de contact est disposé à l'intérieur du corps d'étui et le couvercle fixé à l'extrémité ouverte de ce corps d'étui, l'élément catalytique peut se trouver, soit au-dessus des lentilles de contact à désinfecter, soit en face de celles-ci. De préférence, l'élément catalytique sera disposé de manière à se trouver en face des lentilles de contact afin d'assurer une meilleure circulation de la solution de désinfection autour des lentilles de contact .

Le support de lentilles de contact, selon l'invention, est conçu pour déplacer un volume déterminé de la solution de désinfection lorsqu'il est introduit, jusqu'à une position finale, dans le corps d'étui.

Dans une réalisation recommandée, le support de lentilles de contact est pourvu à son extrémité inférieure d'un piston dimensionné pour assurer une partie du déplacement de la solution de désinfection.

Dans d'autres réalisations, le volume de solution de désinfection déplacé est déterminé par la conception des éléments classiques du support de lentilles de contact sans qu'il soit nécessaire d'y ajouter un piston.

Les solutions oxydantes de désinfection à base de peroxyde d'hydrogène sont bien connues dans la technique et sont décrites entre autre dans la demande de brevet français n° 7624837.

Les catalyseurs de neutralisation des solutions oxydantes à base de peroxyde d'hydrogène sont également bien connus dans la technique.

On peut utiliser dans la présente invention tous catalyseurs de neutralisation des solutions de désinfection, par exemple à base de peroxyde d'hydrogène. Parmi les catalyseurs recommandés, utiles dans la présente invention, on peut citer les métaux du groupe du platine, tels que le platine et le palladium, en particulier le noir de platine, et les enzymes ou catalases immobilisées sur un support, par exemple comme décrit dans les documents EP-A-209 071 et WO-86 07 264.

L'élément catalytique peut être constitué par un dépôt direct de catalyseur sur la surface intérieure de la paroi latérale du corps d'étui, à l'endroit voulu. L'élément catalytique est de préférence constitué par une couche de catalyseur disposée sur une surface d'un substrat, par exemple un substrat en matière plastique, l'élément catalytique étant ensuite fixé à la surface intérieure de la paroi latérale du corps d'étui à l'endroit voulu au moyen de la surface du substrat ne comportant pas la couche de catalyseur. La fixation de l'élément catalytique sur la surface intérieure de la paroi latérale du corps d'étui peut s'effectuer mécaniquement, par exemple par emmanchement à force ou de manière élastique, ou par collage.

Lorsque le catalyseur de l'élément catalytique est constitué par un métal du groupe du platine, il peut être déposé par dépôt chimique en phase vapeur ou par pulvérisation cathodique comme cela est bien connu.

De préférence, l'élément catalytique est constitué par une couche de catalyseur déposée sur la surface interne d'un substrat moulé en matière plastique, par exemple sous la forme d'un anneau fendu. Le substrat de l'élément catalytique est alors fixé à l'endroit voulu sur la surface interne de la paroi latérale du corps d'étui par l'effet de ressort du substrat. On peut également prévoir sur la surface interne de la paroi latérale du corps d'étui des éléments d'enclipsage pour maintenir le substrat revêtu de catalyseur.

De préférence, encore, le substrat en matière plastique comporte à son extrémité supérieure un élément de protection de la couche de catalyseur, tel qu'une saillie transversale recouvrant le catalyseur et dont la surface externe peut être inclinée ou arrondie intérieurement de manière à permettre une introduction plus aisée du support de lentilles de contact dans le corps d'étui. Cet élément de protection peut efficacement aider à son démontage en vue de sa récupération ou de son interchangeabilité.

Enfin, la surface interne de la paroi latérale du corps d'étui destinée à recevoir directement le catalyseur ou la surface interne du substrat destinée à recevoir la couche de catalyseur, peut être pourvue de moyens d'augmentation de la surface de contact entre le catalyseur et la solution de désinfection, tels que des dents, rainures ou picots.

Comme indiqué précédemment, on prévoit sur le corps d'étui un repère visuel disposé en dessous de l'élément catalytique et définissant un volume initial déterminé de solution de désinfection devant être introduit dans le corps d'étui. Un tel repère visuel peut être aisément constitué par une ou deux lignes opaques ou de couleur ou encore par une ou plusieurs saillies ou rainures ménagées dans la paroi latérale du corps d'étui. Ce repère visuel peut être ménagé sur la surface interne ou externe de la paroi latérale du corps d'étui, pour autant qu'il soit aisément visible pour l'utilisateur. Ce repère visuel peut être prévu sur la totalité du périmètre de la paroi latérale ou peut être constitué simplement d'éléments discontinus ménagés sur cette paroi.

Il est important que le repère visuel soit disposé légèrement en dessous de l'extrémité inférieure de l'élément catalytique de façon à s'assurer que lors du remplissage initial du corps d'étui avec la solution de désinfection, le volume de solution introduit soit tel que la solution n'entre pas en contact avec le catalyseur de l'élément catalytique.

D'autre part, ce repère visuel est disposé à une distance telle du fond du corps d'étui qu'il définit un volume de solution de désinfection initial déterminé. Ce volume initial déterminé, défini par le repère visuel, et par conséquent la position du repère visuel en dessous de l'élément catalytique, est fonction du volume du corps d'étui en dessous de l'élément catalytique et des paramètres structuraux du support de lentilles de contact définissant le volume de solution de désinfection déplacé par le support de lentilles de contact lorsqu'il est disposé à l'intérieur du corps d'étui. Ce volume initial déterminé de solution de désinfection doit être suffisant, lorsque le support de lentilles de contact est dans sa position finale à l'intérieur du corps d'étui, pour que le volume de solution de désinfection déplacé par le support de lentilles de contact soit tel que le niveau de la solution de désinfection s'élève jusqu'à ce que la solution recouvre, de préférence entièrement, l'élément catalytique et les lentilles de contact à désinfecter contenues dans le support de lentilles de contact.

Lorsque le repère visuel comporte deux lignes opaques ou de couleur, rainures ou saillies, ces deux lignes, rainures ou saillies, définissent entre elles un intervalle étroit à l'intérieur duquel doit se situer le niveau supérieur du volume initial de solution de désinfection introduit par l'utilisateur. Ainsi, l'utilisateur peut aisément et de façon sûre introduire le volume initial déterminé suffisant de solution de désinfection sans risque de contact avec l'élément catalytique.

Lorsque l'utilisateur veut effectuer la désinfection de lentilles de contact, il introduit initialement dans le corps d'étui un volume déterminé de solution de désinfection, par exemple une solution oxydante à base de peroxyde d'hydrogène. Ce volume déterminé peut être obtenu soit en introduisant une quantité prédéterminée de solution soit en remplissant le corps d'étui jusqu'à un niveau correspondant au repère visuel. Ainsi, la solution de désinfection initialement introduite n'entre pas en contact avec le catalyseur de l'élément catalytique. Il place alors les lentilles de contact à désinfecter dans le support de lentilles de contact puis introduit le support dans le corps d'étui et fixe le couvercle sur l'extrémité ouverte du corps d'étui. Lors de cette introduction, une partie du support de lentilles de contact va déplacer un volume déterminé de solution de désinfection.

Le support de lentilles de contact, comme indiqué précédemment, est conçu pour que le volume de solution de désinfection déplacé soit tel que lorsque le support se trouve fixé dans sa position finale à l'intérieur du corps d'étui, le niveau de la solution s'élève à l'intérieur du corps d'étui jusqu'à recouvrir le catalyseur de l'élément catalytique et les lentilles de contact à désinfecter. Dès que la solution de désinfection se trouve au contact du catalyseur, la réaction de neutralisation commence en même temps que la solution baigne les lentilles de contact dans le support de lentilles de contact et les désinfecte. Une fois les lentilles de contact désinfectées et la solution neutralisée, l'utilisateur enlève le couvercle du corps d'étui et retire le support de lentilles de contact avec les lentilles de contact désinfectées.

Du fait qu'initialement, le volume de solution introduit est tel que le niveau supérieur de la solution se trouve légèrement en dessous de l'extrémité inférieure de l'élément catalytique, la neutralisation de la solution ne peut pas débuter avant que le support de lentilles de contact contenant les lentilles de contact à désinfecter soit immergé dans la solution de désinfection. Ainsi, on évite tout risque que la solution soit neutralisée, avant immersion des lentilles de contact à désinfecter, à un point tel qu'il en résulte une désinfection défectueuse.

D'autre part, l'élément catalytique se trouvant sur la surface intérieure de la paroi latérale du corps d'étui de l'ensemble de désinfection, on évite un risque de contact accidentel du catalyseur avec les doigts de l'utilisateur. En outre, lorsque l'élément catalytique est constitué par un substrat dont une surface est revêtue du catalyseur et dont l'extrémité supérieure est pourvue d'un élément de protection, on évite également tout risque de détérioration du catalyseur par contact avec le support de lentilles de contact lors de l'introduction de celui-ci dans le corps d'étui. Lorsque l'élément de protection comporte une surface supérieure inclinée ou arrondie vers l'intérieur, il facilite également le centrage du support de lentilles de contact lors de son introduction.

La présente invention concerne également un procédé de désinfection de lentilles de contact au moyen d'un système de désinfection qui consiste à :
- fournir un étui constitué d'un corps d'étui, de préférence transparent ou translucide, ouvert à une extrémité, ayant une paroi de fond et une paroi latérale, et d'un couvercle amovible destiné à être fixé sur l'extrémité ouverte du corps d'étui;
- un support de lentilles de contact destiné à être introduit à l'intérieur du corps d'étui jusqu'à une position finale; et
- un élément catalytique de neutralisation de la solution oxydante disposé sur la surface interne de la paroi latérale du corps d'étui à une distance prédéterminée de la paroi de fond du corps d'étui;
- remplir le corps d'étui de l'ensemble de désinfection avec un volume déterminé d'une solution de désinfection, par exemple une solution oxydante à base de peroxyde d'hydrogène, de telle sorte qu'initialement le niveau supérieur de la solution dans le corps d'étui soit situé en dessous de l'extrémité inférieure de l'élément catalytique;
- introduire le support de lentilles de contact avec les lentilles de contact à désinfecter dans le corps d'étui jusqu'à sa position finale; et, ainsi déplacer un volume prédéterminé de la solution de désinfection initialement introduite, de telle sorte que la solution recouvre l'élément catalytique et les lentilles de contact à désinfecter pour ainsi effectuer simultanément la désinfection des lentilles de contact et la neutralisation de la solution de désinfection.

De préférence, le support de lentilles de contact est solidaire du couvercle et saille à partir de ce couvercle de manière à pénétrer à l'intérieur du corps d'étui, jusqu'à sa position finale, lorsque le couvercle est fixé sur l'extrémité ouverte du corps d'étui.

De préférence également, le volume de solution de désinfection déplacé est tel que cette solution recouvre entièrement l'élément catalytique et les lentilles de contact lorsque le support de lentilles de contact se trouve dans sa position finale à l'intérieur de l'étui.

La suite de la description se réfère aux figures annexées qui représentent, respectivement :
figure 1 - une vue schématique d'une première réalisation d'un ensemble de désinfection de lentilles de contact selon l'invention;
figure 2 - une vue schématique d'une seconde réalisation d'un ensemble de désinfection de lentilles de contact selon l'invention;
figure 3 - une vue schématique d'une troisième réalisation d'un ensemble de désinfection de lentilles de contact selon l'invention;
figure 4 - une vue schématique d'une quatrième réalisation d'un ensemble de désinfection de lentilles de contact selon l'invention;
figure 5 - une vue schématique d'une cinquième réalisation d'un ensemble de désinfection de lentilles selon l'invention dans lequel le support de lentilles est réalisé d'une seule pièce avec le couvercle;
figure 6 - une vue de côté du couvercle et du support de lentilles monobloc de la réalisation de la figure 5 avec les paniers du support de lentilles en position ouverte;
figure 7 - une vue en coupe des paniers du support de lentilles de la réalisation de la figure 5 dans leur position fermée; et
figure 8 - une vue en coupe faite suivant la ligne a-a de la figure 5, avec un panier du support de lentilles en position ouverte et montrant le dispositif d'articulation et le dispositif de verrouillage du panier.

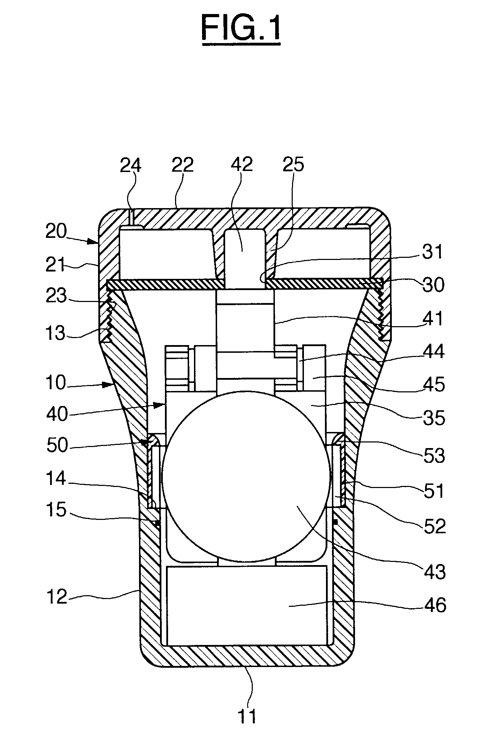

En se référant aux figures, où les mêmes numéros de référence correspondent aux mêmes éléments, et en particulier à la figure 1, on a représenté une première réalisation d'un ensemble de désinfection de lentilles de contact selon l'invention.

L'ensemble comprend un corps d'étui 10 de forme générale cylindrique, un couvercle amovible 20 s'adaptant sur une extrémité ouverte du corps d'étui, un support de lentilles de contact 40 et un élément catalytique 50.

Comme on le voit sur la figure 1, le corps d'étui 10 comprend une paroi latérale 12 de forme générale cylindrique s'évasant vers l'extérieur en direction de son extrémité ouverte et une paroi de fond 11. Bien évidemment, le corps d'étui 10 pourrait être de toute autre forme appropriée, telle qu'une forme polygonale, ovale, etc...

La paroi latérale cylindrique 12 du corps d'étui est généralement pourvue extérieurement à son extrémité supérieure ouverte d'un filetage 13 pour la connexion au couvercle. Toutefois, on peut utiliser tout autre moyen de réunion du couvercle 20 au corps d'étui 10, tel qu'un emmanchement serré, un enclipsage, etc...

La paroi latérale 12 du corps d'étui est également pourvue intérieurement à une distance prédéterminée de la paroi de fond 11 d'un rebord annulaire 14 dont la fonction sera expliquée dans la suite.

Enfin, la surface intérieure de la paroi latérale 12 du corps d'étui est munie d'un repère visuel 15, par exemple une ligne de coloration différente, une saillie ou une rainure, disposé très légèrement en dessous du rebord annulaire interne 14 et définissant un volume déterminé.

Généralement, le corps d'étui 10 est une pièce moulée en matière plastique, de préférence transparente ou translucide, par exemple en polystyrène cristal.

L'extrémité supérieure ouverte du corps d'étui 10 est fermée par un couvercle 20 de forme générale cylindrique dont la paroi latérale 21 comporte près de son extrémité ouverte un filetage interne 23 coopérant avec le filetage 13 ménagé extérieurement sur la paroi latérale 12 du corps d'étui 10 près de l'extrémité ouverte de celui-ci pour fixer le couvercle au corps d'étui. Le couvercle 20 comporte également au moins un orifice de dégazage 24 ménagé dans sa paroi de fond.

Le couvercle 20 est également muni en son centre d'un logement cylindrique 25 faisant saillie intérieurement à partir de sa paroi de fond 22. La fonction de ce logement 22 sera expliquée dans la suite de la description.

Comme on le voit sur la figure 1, la paroi latérale cylindrique 21 du couvercle comporte un rebord annulaire intérieur près de son extrémité ouverte, et un joint annulaire 30 pourvu d'un trou central 31 est disposé généralement entre le rebord annulaire intérieur du couvercle et l'extrémité supérieure ouverte du corps d'étui 10. Un orifice, tel qu'une fente en I ou un prétrou (non représenté) est généralement prévu dans le joint 30 pour permettre le dégazage lors de la désinfection des lentilles de contact.

De préférence, le couvercle 20 est constitué d'une seule pièce moulée en matière plastique, par exemple en polypropylène, et le joint 30 est généralement un joint en silicone. On peut également utiliser des joints constitués d'autres matériaux permettant d'assurer l'étanchéité, tels que le polybutadiène.

L'ensemble de désinfection comprend également un support de lentilles de contact 40. Le support de lentilles de contact 40 comprend un axe central vertical de support 41 pourvu à son extrémité supérieure d'une saillie cylindrique 42 destinée à venir se loger dans le logement cylindrique 25 du couvercle 20 de façon à solidariser le support de lentilles de contact 40 au couvercle 20. Cette solidarisation peut s'effectuer, soit par emmanchement en force de la saillie cylindrique 42 dans le logement cylindrique 25, par enclipsage, soit encore par collage de cette saillie cylindrique 42 dans le logement cylindrique 25. Comme on le voit également sur la figure 1, le trou central 31 du joint d'étanchéité 30 est dimensionné pour permettre le passage de la saillie cylindrique 42 de l'axe central vertical 41 du support de lentilles de contact 40.

Les lentilles de contact sont maintenues dans le support de lentilles de contact 40 entre des surfaces convexes opposées ménagées sur l'axe 41 et par une paire de paniers convexes 35 articulés autour d'un axe transversal 44 fixé à la partie supérieure de l'axe central vertical 41 par des pattes de pivotement 45. Le support de lentilles de contact 40 que l'on vient de décrire est classique et on pourra se référer pour plus de détails quant aux différentes réalisations d'un tel support de lentilles de contact, par exemple aux brevets des Etats-Unis 4 396 583 et 4 637 919, et au brevet européen EP 354 876.

Comme le montre la figure 1, le support de lentilles de contact 40 comporte en outre un piston cylindrique 46 fixé à l'extrémité inférieure de l'axe vertical central 41 en dessous des paniers 43.

L'ensemble de désinfection comprend un élément catalytique 50 disposé le long d'une partie de la surface intérieure de la paroi latérale 12 du corps d'étui 10 et reposant sur le rebord 14. Cet élément catalytique 50 comprend un substrat 51 de forme générale annulaire, en matière plastique, par exemple en polyoxyde de phénylène, dont la surface interne est recouverte d'une couche 52 d'un matériau catalytique actif pour la neutralisation de la solution oxydante à base de peroxyde d'hydrogène, tel qu'un métal du groupe du platine, par exemple le platine ou le palladium ou encore une enzyme immobilisée sur un support. Comme le montre la figure 1, l'extrémité supérieure du substrat en matière plastique 51 comporte une saillie annulaire 53 orientée intérieurement qui sert à protéger la couche 52 de catalyseur sur la surface interne du substrat 51 de tout contact accidentel, en particulier avec le support de lentilles de contact 40 lors de l'introduction de celui-ci. De préférence, également, comme le montre la figure 1, la surface supérieure de cette saillie 53 est courbe et inclinée vers l'intérieur de façon à faciliter l'introduction et le guidage du support de lentilles de contact 40 dans le corps d'étui 10 de l'ensemble de désinfection.

Comme on le voit sur la figure 1, le rebord annulaire intérieur 14 de la paroi latérale 12 du corps d'étui 10 sur lequel vient reposer l'élément catalytique 50 est à une distance prédéterminée telle de la paroi de fond 11 du corps d'étui que l'élément catalytique 50 se trouve disposé en face des paniers de réception de lentilles de contact 43, et par conséquent des lentilles de contact qui y sont contenues, lorsque le couvercle est fixé sur le corps d'étui et le support de lentilles de contact 40 disposé à l'intérieur du corps d'étui.

On va maintenant décrire le fonctionnement de l'ensemble de désinfection selon l'invention, représenté à la figure 1.

Lorsque l'utilisateur souhaite désinfecter des lentilles de contact, il dévisse le couvercle 20 pour le séparer du corps d'étui 10 en même temps que le support de lentilles de contact 40. Il introduit alors dans le corps d'étui 10 une solution de désinfection, par exemple à base de peroxyde d'hydrogène, de façon à remplir le corps d'étui jusqu'à ce que le niveau de la solution atteigne ou soit légèrement inférieur au repère visuel 15. L'utilisateur introduit ainsi un volume initial déterminé de solution de désinfection. Comme le repère visuel se trouve légèrement en dessous de l'extrémité inférieure de l'élément catalytique 50, à ce stade, la solution de désinfection ne se trouve pas en contact avec la couche de catalyseur 52 de l'élément catalytique. L'utilisateur place alors les lentilles de contact à désinfecter dans les paniers 43 du support de lentilles de contact 40, puis il introduit le support 40 dans le corps d'étui 10 en revissant le bouchon 20 sur l'extrémité ouverte du corps d'étui. Lors de cette étape, le piston 46 et une partie des paniers 43 sont introduits dans la solution oxydante contenue dans le corps d'étui 10 et déplacent une partie du volume de solution de désinfection, élevant ainsi le niveau de la solution. Le piston 46 et les paniers 43 du support de lentilles de contact sont conçus de telle sorte et le volume initial déterminé introduit de solution oxydante est tel que le volume déplacé de solution lors de l'introduction du support de lentilles de contact dans le corps d'étui élève le niveau de la solution à l'intérieur du corps d'étui 10 jusqu'à ce que la solution recouvre, de préférence totalement, l'élément catalytique 50 et les lentilles de contact contenues dans le support de lentilles de contact 40. Dès que la solution entre en contact avec le catalyseur 52 de l'élément catalytique 50, la neutralisation de la solution débute. Ainsi, la neutralisation de la solution a lieu en même temps que s'effectue la désinfection des lentilles de contact au moyen de cette solution. D'autre part, la neutralisation de la solution ne commence qu'au moment où les lentilles de contact sont immergées dans cette solution, et par conséquent on assure ainsi que la solution n'a pas été neutralisée, avant le début de la désinfection, à un point tel que la désinfection serait défectueuse.

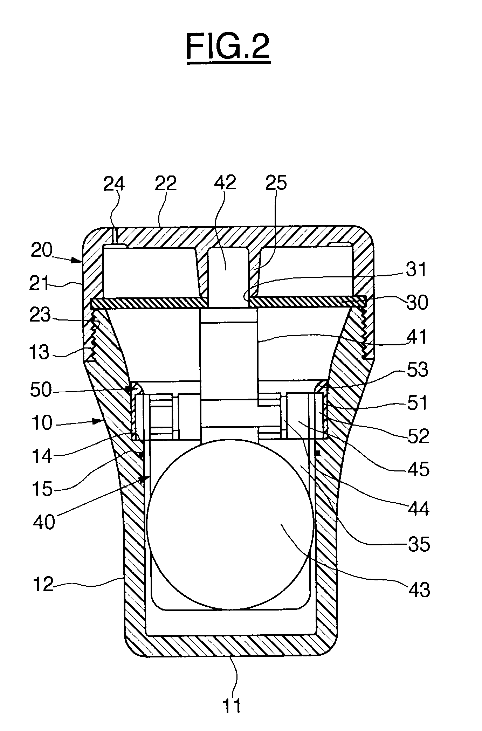

On a représenté sur la figure 2 une deuxième réalisation d'un ensemble de désinfection selon l'invention qui ne diffère de la réalisation de la figure 1 qu'en ce que le support de lentilles de contact 40 ne comporte pas à son extrémité inférieure de piston cylindrique 46 et en ce que le rebord annulaire interne 14 de la paroi latérale 12 du corps d'étui 10 se trouve à une distance prédéterminée de la paroi de fond 11 du corps d'étui 10 telle que l'élément catalytique 50 reposant sur ce rebord 14 se trouve situé au-dessus des paniers de réception de lentilles de contact 43 lorsque le support de lentilles de contact 40 est introduit dans le corps d'étui 10 et le couvercle 20 vissé sur l'extrémité ouverte du corps d'étui.

Dans cette réalisation, bien évidemment, le support de lentilles de contact est conçu pour, lors de son introduction, déplacer un volume de solution tel que le niveau de la solution dans le corps d'étui 10 s'élève jusqu'à recouvrir l'élément catalytique 50. L'ensemble de désinfection de la figure 2 fonctionne de la même façon que le système de la figure 1.

Bien que l'on ait représenté dans les réalisations des figures 1 et 2 un piston 46 allant jusqu'à la paroi de fond 11 du corps d'étui 10, le piston peut être conçu de manière à ménager un espace avec la paroi de fond 11, la seule condition étant que le volume de solution déplacé soit le volume nécessaire pour recouvrir l'élément catalytique 50.

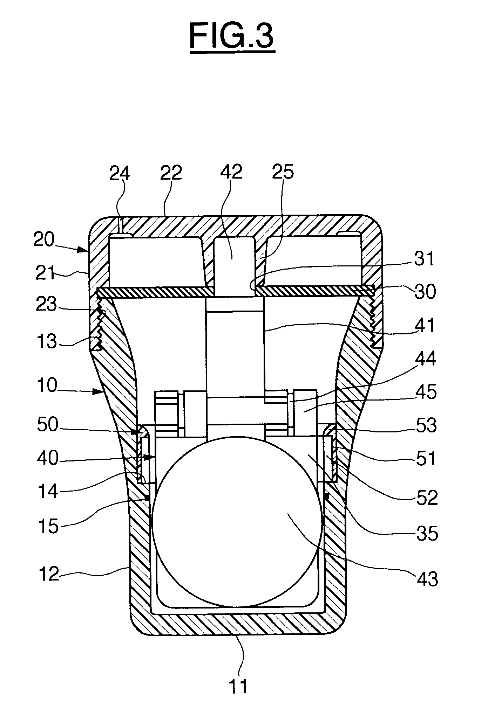

On a représenté à la figure 3 une troisième réalisation d'un ensemble de désinfection selon l'invention qui ne diffère du système de la figure 2 qu'en ce que le rebord annulaire interne 14 sur la paroi latérale 12 du corps d'étui 10 se trouve à une distance prédéterminée de la paroi de fond 11 telle que l'élément catalytique 50 qu'il supporte se trouve situé au niveau de la partie supérieure des paniers de lentilles 43. En disposant ainsi le rebord annulaire interne 14 et l'élément catalytique 50, on utilise un volume moindre de solution oxydante que dans l'ensemble de désinfection de la figure 2. Le fonctionnement de l'ensemble de désinfection représenté à la figure 3 est analogue à ceux des réalisations précédentes.

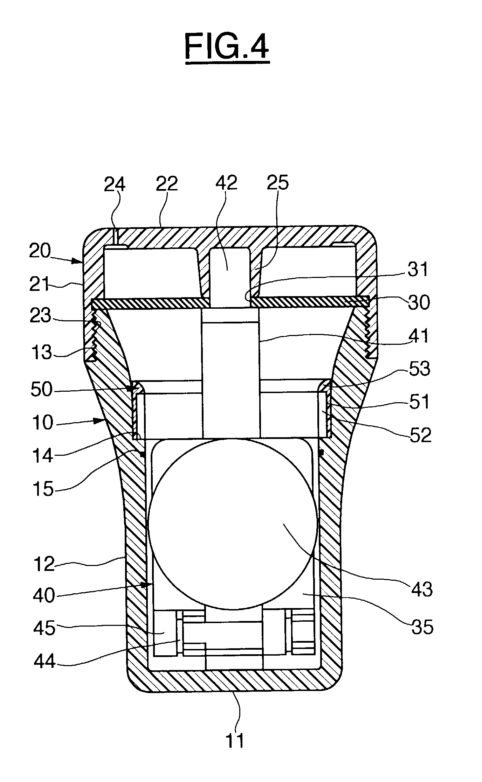

On a représenté à la figure 4 une quatrième réalisation d'un ensemble de désinfection de l'invention analogue à celui de la figure 2 et qui ne diffère de celui-ci qu'en ce que l'axe transversal d'articulation 44 des paniers de réception de lentilles de contact 43 est disposé à l'extrémité inférieure de l'axe central vertical 41 du support de lentilles de contact 40. Avec une telle disposition, l'axe transversal 44 d'articulation joue un rôle analogue au piston 46 de la réalisation de la figure 1 pour le déplacement de la solution de désinfection. L'ensemble de désinfection de lentilles de contact de cette quatrième réalisation fonctionne également comme les réalisations précédentes.

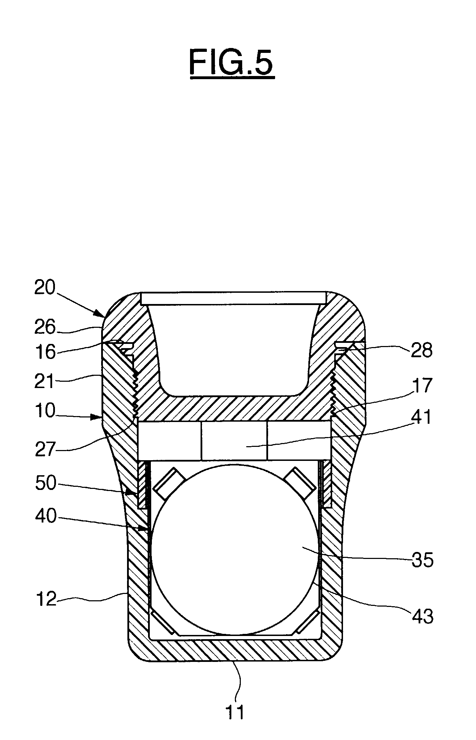

On a représenté à la figure 5 une autre réalisation d'un ensemble de désinfection selon l'invention dans lequel le couvercle 20 et le support de lentilles de contact 40 forment un sous-ensemble monobloc, par exemple une seule pièce en matière plastique telle que du polypropylène, moulée par injection.

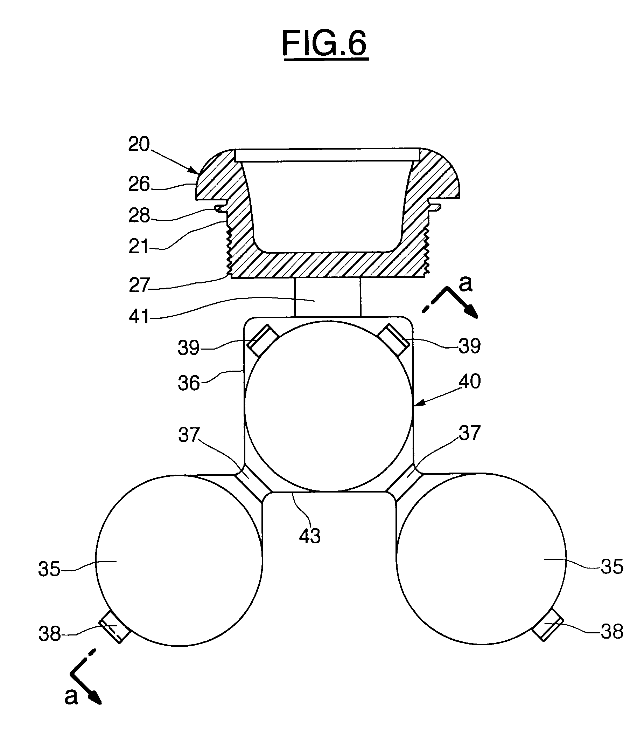

Plus précisément, en se référant aux figures 5 et 6, le couvercle 20 est conçu pour être fixé sur l'extrémité ouverte du corps d'étui, par exemple par vissage ou emboîtement à l'intérieur de l'extrémité ouverte du corps d'étui 10. Dans la réalisation représentée, le couvercle est vissé. Le couvercle 20 est de forme générale cylindrique et comporte à son extrémité supérieure un rebord annulaire 26 destiné à venir s'appuyer sur la paroi latérale cylindrique 21 du corps d'étui 10 à son extrémité ouverte pour constituer un premier élément d'étanchéité. La surface latérale du couvercle 20 est munie à son extrémité inférieure d'un redent annulaire 27 destiné à venir en butée sur une saillie annulaire 17 ménagée dans la surface interne de la paroi latérale 21 du corps d'étui. La venue en butée du redent annulaire 27 sur la saillie annulaire 17 réalise un deuxième élément d'étanchéité.

Une lèvre annulaire souple 28 est ménagée sur la paroi latérale cylindrique du couvercle en dessous du rebord annulaire 26. Lorsque le bouchon est enfoncé dans le corps d'étui, cette lèvre annulaire souple 28 vient s'appuyer sur une surface annulaire chanfreinée 16 ménagée dans la paroi latérale du corps d'étui 10 à son extrémité ouverte. La lèvre annulaire souple 28 joue ainsi le rôle d'un joint d'étanchéité. En outre, du fait de sa souplesse, cette lèvre 28 joue également le rôle d'un dispositif de dégazage. Sous l'effet de la pression du gaz qui se dégage lors de la réaction de neutralisation, cette lèvre 28 s'écarte de la surface d'appui inclinée 16, laissant le gaz s'échapper et évitant ainsi l'établissement d'une surpression à l'intérieur de l'ensemble.

On peut, à la place de la lèvre souple 28 utiliser un joint torique, mais cette solution est moins recommandée car elle nécessite une pièce supplémentaire.

Le support de lentilles 40 d'une seule pièce avec le couvercle 20 comprend des paniers de réception de lentilles 43 reliés au couvercle 20 par un axe 41. Ces paniers de réception de lentilles 43 comprennent un cadre 36, ayant la forme générale d'un quadrilatère pourvu d'une ouverture centrale circulaire, relié par un côté à l'axe 41.

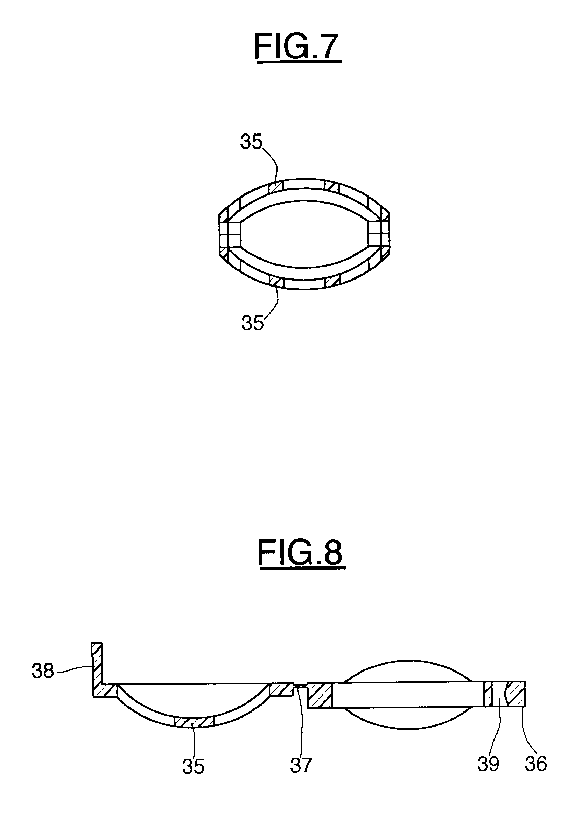

Aux angles du côté du cadre 36 opposé au côté solidaire de l'axe 41, sont fixés par des charnières 37 deux paniers convexes 35. Ces paniers 35 sont disposés en sens inverse l'un de l'autre pour que, comme le montre la figure 7, lorsque le support de lentilles est fermé, les paniers pivotants 35 soient disposés contre les faces opposées du cadre 36.

En se référant également à la figure 8, on va maintenant décrire les moyens de verrouillage des paniers 35 en position fermée sur le cadre 36 ainsi qu'une réalisation recommandée des charnières 37 permettant le pivotement des paniers.

Comme représenté, les paniers 35 comportent à leur périphérie, à l'opposé des charnières 37, un languette en fermeture 38, cependant que des ouvertures correspondantes 39 sont ménagées dans les coins du cadre 36 opposés aux paniers 35. Comme on le voit plus précisément sur la figure 8, les languettes 38 comportent un picot terminal et les ouvertures 39 comportent sur leur surface interne une saillie qui coopèrent pour bloquer par encliquetage les paniers 35 sur le cadre 36.

Comme on le voit également sur la figure 8, les charnières 37 sont de préférence formées par une partie étroite amincie réunissant les paniers 35 au cadre 36, par exemple une simple diminution de l'épaisseur du matériau polymère utilisé pour fabriquer le support de lentilles 40.

Pour le reste, l'ensemble de désinfection de la figure 5 est semblable à la réalisation de la figure 2 et fonctionne de manière analogue.

Bien évidemment, le couvercle 20 et le support de lentilles monobloc de la réalisation de la figure 5 peuvent être utilisés dans d'autres ensembles de désinfection, en particulier ne comprenant pas un effet de piston comme dans la présente invention.

Afin de réduire la concentration en péroxyde résiduel en fin de neutralisation, on peut prévoir un moyen, situé dans la partie inférieure du corps d'étui, de préférence placé sur la paroi de fond 11 de ce corps d'étui, permettant une agitation de la solution lors de la phase de décontamination/neutralisation.

Ainsi, le péroxyde d'hydrogène non encore neutralisé pourra plus facilement accéder à la partie supérieure de l'étui pour y entrer en contact avec le catalyseur.

On peut prévoir comme moyen d'agitation, par exemple, une petite pastille de catalyseur maintenue sur la paroi de fond 11 du corps de l'étui. Le dégagement d'oxygène permettra de véhiculer le péroxyde d'hydrogène vers la partie supérieure du corps d'étui. On peut aussi prévoir un dépôt de catalyseur sur la paroi de fond 11 du corps de l'étui.

Dans tous les cas, les quantités et/ou l'activité du catalyseur situé en partie basse de l'étui sont très inférieures à celles de l'élément catalytique 50 situé sur la partie supérieure du corps de l'étui où s'effectue l'essentiel de la réaction de neutralisation.

A la place du catalyseur comme moyen d'agitation, on peut utiliser un produit n'ayant pas d'effet catalytique mais susceptible de dégager des bulles au contact de la solution de péroxyde.

Enfin, les différents moyens d'agitation décrits précédemment peuvent être fixés sur la partie inférieure du support de lentilles 40.

## Revendications

1. Ensemble pour la désinfection de lentilles de contact au moyen d'une solution de désinfection, comprenant :
- un étui constitué d'un corps d'étui (10) ouvert à une extrémité, ayant une paroi de fond (11) et une paroi latérale (12), et d'un couvercle (20) amovible destiné à être fixé sur l'extrémité ouverte du corps d'étui;
- un support de lentilles de contact (40) destiné à être introduit jusqu'à une position finale à l'intérieur du corps d'étui (10); et
- un élément catalytique (50) de neutralisation de la solution de désinfection disposé sur la surface interne de la paroi latérale (12) du corps d'étui à une distance prédéterminée de la paroi de fond (11) du corps d'étui (10), caractérisé en ce qu'il comprend en outre un repère visuel (15) sur la paroi latérale (12) du corps d'étui (10) disposé à une distance prédéterminée en dessous de l'élément catalytique (50) et définissant un volume prédéterminé de solution de désinfection à introduire initialement dans le corps d'étui (10), et en ce que le support de lentilles de contact (40) est conçu de telle sorte que lorsque le support de lentilles de contact (40) introduit dans le corps d'étui (10) est en position finale, le volume de solution de désinfection déplacé par le support de lentilles de contact est suffisant pour recouvrir l'élément catalytique (50) et les lentilles de contact à désinfecter.

2. Ensemble selon la revendication 1, caractérisé en ce que l'élément catalytique (50) se trouve situé au-dessus des lentilles de contact lorsque le support de lentilles de contact (40) est disposé à l'intérieur du corps d'étui (10).

3. Ensemble selon la revendication 1, caractérisé en ce que l'élément catalytique (50) est situé en face des lentilles de contact lorsque le support de lentilles de contact (40) est disposé à l'intérieur du corps d'étui (10).

4. Ensemble selon l'une quelconque des revendications précédentes, caractérisé en ce que l'élément catalytique (50) comprend une couche de catalyseur déposée directement sur une partie de la surface interne de la paroi latérale (12) du corps d'étui (10).

5. Ensemble selon l'une quelconque des revendications 1 à 3, caractérisé en ce que l'élément catalytique (50) comprend un substrat (51) dont la surface interne est revêtue d'une couche de catalyseur (52).

6. Ensemble selon la revendication 5, caractérisé en ce que le substrat (51) comporte un élément de protection (53) du catalyseur (52).

7. Ensemble selon la revendication 6, caractérisé en ce que l'élément de protection (53) est une saillie transversale formée à l'extrémité supérieure du substrat.

8. Ensemble selon la revendication 7, caractérisé en ce que la saillie transversale comporte une surface externe inclinée arrondie intérieurement.

9. Ensemble selon l'une quelconque des revendications 5 à 8 caractérisé en ce que la surface du substrat (51) revêtue de la couche de catalyseur (52) comporte des moyens augmentant la surface de la couche de catalyseur (52).

10. Ensemble selon la revendication 9, caractérisé en ce que les moyens d'augmentation de la surface de la couche de catalyseur sont constitués par des dents, des rainures ou des picots.

11. Ensemble selon l'une quelconque des revendications précédentes, caractérisé en ce que le support de lentilles de contact (40) comporte à son extrémité inférieure un piston (46) assurant une partie du déplacement de la solution de désinfection.

12. Ensemble selon l'une quelconque des revendications précédentes, caractérisé en ce que le corps d'étui (10) est transparent ou translucide.

13. Ensemble selon l'une quelconque des revendications précédentes, caractérisé en ce que le support de lentilles de contact (40) est solidaire du couvercle (20) et saille à partir de ce couvercle de manière à pénétrer à l'intérieur du corps d'étui (10), jusqu'à sa position finale, lorsque le couvercle est fixé sur l'extrémité ouverte du corps d'étui.

14. Ensemble selon la revendication 1, caractérisé en ce que le couvercle (20) comporte une lèvre annulaire souple (28) servant de moyen de dégazage de l'ensemble de désinfection.

15. Ensemble selon l'une quelconque des revendications précédentes, caractérisé en ce que le couvercle (20) et le support de lentilles (40) forment un sous-ensemble monobloc.

16. Procédé de désinfection de lentilles de contact, caractérisé en ce qu'il consiste à :
- fournir un étui constitué d'un corps d'étui (10) ouvert à une extrémité, ayant une paroi de fond (11) et une paroi latérale (12), et d'un couvercle (20) amovible destiné à être fixé sur l'extrémité ouverte du corps d'étui;
- un support de lentilles de contact (40) destiné à être introduit à l'intérieur du corps d'étui (10) jusqu'à une position finale; et
- un élément catalytique (50) de neutralisation de la solution de désinfection disposé sur la surface interne de la paroi latérale (12) du corps d'étui à une distance prédéterminée de la paroi de fond (11) du corps d'étui (10);
- remplir le corps d'étui (10) avec un volume déterminé d'une solution de désinfection de telle sorte que le niveau de la solution dans le corps d'étui se trouve initialement en dessous de l'élément catalytique (50);
- introduire le support de lentilles de contact (40) contenant des lentilles de contact à désinfecter dans le corps d'étui (10) jusqu'à sa position finale, et ainsi déplacer un volume prédéterminé de la solution de désinfection initialement introduite, de telle sorte que la solution de désinfection recouvre l'élément catalytique (50) et les lentilles de contact à désinfecter pour effectuer simultanément la désinfection des lentilles de contact et la neutralisation de la solution de désinfection.

17. Procédé selon la revendication 16, caractérisé en ce que le support de lentilles de contact (40) est solidaire du couvercle (20) et saille à partir de ce couvercle de manière à pénétrer à l'intérieur du corps d'étui (10) lorsque le couvercle est fixé sur l'extrémité ouverte du corps d'étui.
